# EUROPÄISCHE PATENTSCHRIFT

(11) **EP 0 653 243 B1**
(45) Veröffentlichungstag und Bekanntmachung des Hinweises auf die Patenterteilung: **31.01.2001**
(21) Anmeldenummer: 94117574.7
(22) Anmeldetag: 08.11.1994
(51) Int. Cl.: B01J 37/00, B01J 37/02, B01J 23/44, C07C 5/09, C07C 5/05, C10G 45/34

(54) **Trägerkatalysatoren**
Supported catalysts
Catalyseurs supportés

(30) Priorität: 16.11.1993 DE 4339138
(43) Veröffentlichungstag der Anmeldung: 17.05.1995
(73) Patentinhaber: BASF Aktiengesellschaft, 67063 Ludwigshafen (DE)
(72) Erfinder: Flick, Klemens, Dr., D-76863 Herxheim (DE); Polanek, Peter, Dr., D-69469 Weinheim (DE); Posselt, Dietmar, Dr., D-67069 Ludwigshafen (DE)

(56) Entgegenhaltungen:
- EP-A- 0 569 077
- US-A- 4 762 956
- PATENT ABSTRACTS OF JAPAN vol. 010 no. 123 (C-344) ,8.Mai 1986 & JP-A-60 248237 (NIPPON SHOKUBAI KAGAKU KOGYO KK) 7.Dezember 1985,
- : "RömppChemie Lexicon, Seiten 3587-88", 1992, THIEME VERLAG, STUTTGART

## Beschreibung

Die vorliegende Erfindung betrifft Trägerkatalysatoren, ein Verfahren zu ihrer Herstellung sowie ihre Verwendung.

Die Verteilung der katalytisch aktiven Komponenten in Trägerkatalysatoren spielt für deren Aktivität, Selektivität und Standzeit eine entscheidende Rolle. Werden Trägerkatalysatoren beispielsweise durch Tränkung von porösen Katalysatorträgern mit Lösungen, die die katalytisch aktiven Komponenten oder deren Vorläuferverbindungen enthalten, oder durch gemeinsame Fällung der aktiven Komponenten und des Trägermaterials hergestellt, kommt es zu einer gleichmäßigen Verteilung der aktiven Komponente über den gesamten Katalysator oder zur Ausbildung einer Aktivkomponenten haltigen Schale. Da in der Regel Mikroporen mit einem Durchmesser von < 20 nm den Hauptanteil an der inneren Oberfläche eines Katalysators bilden, befindet sich die aktive Komponente zum größten Teil in diesen Mikroporen. Die Mikroporen sind aber von den Reaktionspartnern schlechter zu erreichen als Meso- und Makroporen (20 bis 100 nm bzw. > 100 nm Durchmesser). Besonders bei diffusionskontrollierten Reaktionen führt das zu einer niedrigen Aktivität und Selektivität, da bevorzugt in den Mikroporen unerwünschte Folgereaktionen ablaufen. Bei katalytischen Reaktionen an sterisch anspruchsvollen Molekülen sinkt der aufgrund der katalytisch wirksamen Menge an aktiven Komponenten im Katalysator mögliche Umsatz bzw. die Aktivität, da diese Moleküle nicht in die Mikroporen eindringen können.

Die JP-A 84/104 678 (Veröffentlichungsnummer JP-A-60 248 237) betrifft ein Verfahren zur Herstellung von Trägerkatalysatoren, wobei eine vernetzte Polyacrylsäure, Wasser, Diatomenerde als Trägermaterial sowie Vanadiumoxid als aktive Komponente vermischt, extrudiert, in Ringe geschnitten und bei 550°C calciniert werden. Der so erhaltene Katalysator zeichne sich durch gute Verarbeitungseigenschaften bei der Extrusion aus.

US-A-4 762 956 lehrt die Verwendung von Palladium-Trägerkatalysatoren auf Aluminiumoxid-Trägern zur selektiven Hydrierung von Acetylenen und Dienen, die als Verunreinigungen in Olefinströmen enthalten sind.

Es bestand die Aufgabe, Trägerkatalysatoren bereitzustellen, deren aktive Komponenten sich zum überwiegenden Teil in Makroporen befinden.

Dementsprechend wurden Trägerkatalysatoren gefunden, die erhältlich sind durch
a) Lösen einer katalytisch aktiven Komponente oder deren Vorläuferverbindung in einem Lösungsmittel,
b) Versetzen der so erhaltenen Lösung mit einem organischen Polymeren, das in der Lage ist, mindestens das Zehnfache seines Eigengewichts an Wasser zu binden,
c) anschließendes Vermischen des Polymeren mit einem Katalysator-Trägermaterial,
d) Formen der so erhaltenen Masse, Trocknen und Calcinieren.

Weiterhin wurde ein Verfahren zur Herstellung dieser Trägerkatalysatoren gefunden sowie ihre Verwendung.

Die erfindungsgemäßen Katalysatoren werden durch die im folgenden beschriebenen Verfahrensschritte hergestellt.

### Verfahrensschritt a)

Die katalytisch aktiven Komponenten oder ihre Vorläuferverbindungen, die erst in weiteren Verarbeitungs- oder Aktivierungsschritten in aktive Komponenten überführt werden, werden in einem Lösungsmittel gelöst. Bevorzugt handelt es sich bei dem Lösungsmittel um polare, mit Wasser mischbare Lösungsmittel wie Alkohole, Ether und Amine. Als Alkohole sind besonders C₁-C₄-Alkohole wie Methanol, Ethanol, iso-Propanol und n-Butanol zu nennen; als Ether kommt z.B. Tetrahydrofuran in Betracht. Erfindungsgemäß zu verwendende Amine sind Ammoniak, Monoamine wie Dimethylamin, Methylamin, Trimethylamin, Ethylamin, Propylamin und Butylamin. Besonders bevorzugt wird aber Wasser oder ein Ammoniak/Wasser-Gemisch.

Die katalytisch aktiven Komponenten sind im allgemeinen wasserlösliche Salze von Übergangsmetallen wie Palladiumnitrat, Palladiumacetat, Ammoniumhexachloropalladat(IV), Ammoniumtetrachloropalladat(II), Dichlorodiamminpalladium(II), Kaliumhexachloropalladat (IV), Kaliumtetrachloropalladat(II), Natriumtetrachloropalladat(II), Palladium-Acetylacetonat, Palladiumchlorid(II), Palladiumsulfat(II), Dichlorotetraamminpalladium(II), Tetraamminpalladiumnitrat, Platinchlorid, Ammoniumhexabromoplatinat, Ammoniumhexachloroplatinat(IV), Ammoniumtetrachloroplatinat(II), Bariumtetracyanoplatinat(II), Hexachloroplatin(IV)-säure, Kaliumhexabromoplatinat(IV), Kaliumhexachloroplatinat(IV), Kaliumtetrachloroplatinat(II), Kaliumtetracyanoplatinat(II), Natriumhexachloroplatinat(IV), Platin(II)-diamminchlorid, Platin(II)-tetraamminchlorid, Platin(II)-tetraamminnitrat; Silbernitrat, -acetat, -sulfat, -cyanid, -carbonat, -rhodamid; Kupfernitrat, -acetat, -sulfat, -chlorid; Zinknitrat, -acetat, -sulfat, -chlorid, -carbonat; Fe(II)-Chlorid, Fe(III)-Chlorid, Eisennitrat, Eisensulfat; Chromchlorid, - sulfat, Kaliumdichromat, Kaliumchromat; Nickelnitrat, -acetat, -sulfat, -chlorid; Mangansulfat, -chlorid, Kaliumpermanganat; Cobaltnitrat, -acetat, -sulfat, -chlorid; Ammoniumhexachlororhodat(III), Natriumhexachlororhodat(III); Rhodium(III)-chlorid, Rhodiumacetat, Rhodium(III)-nitrat, Rhodium(III)-sulfat; Rutheniumnitrat, -acetat, -nitrosylnitrat; Rhenium(VII)-oxid, Ammoniumperrhenat, Natrium- und Kaliumperrhenat, Perrheniumsäure;
Osmiumchlorid, Ammoniumhexachloroosmat, Osmium(VIII)oxid; Kaliumhexachloroosmat(IV), Kaliumosmat(VI).

Diese Verbindungen sind im Handel erhältlich. Weiterhin kommen Sole der Metalle Palladium, Platin, Silber und Kupfer in Betracht, wie sie z.B. nach Angew. Chem. 103 (1991) 852 oder zum Teil im Handel erhältlich sind.

Bevorzugte Verbindungen sind die Salze von Platin, Silber, Nickel, Kupfer und Palladium, besonders bevorzugt Palladiumacetat, -nitrat, -chlorid.

Soll der Trägerkatalysator neben den eigentlich aktiven Komponenten weiterhin Promotoren oder Moderatoren enthalten, die die katalytische Aktivität oder Selektivität beeinflussen können, ist es zweckmäßig, diese direkt oder ebenfalls in Form ihrer Vorläuferverbindungen der Lösung der Übergangsmetallsalze oder -sole zuzusetzen. Im einzelnen sind als Promotoren oder deren Vorläufer zu nennen:

Nitrate, Acetate, Chloride, Sulfate und Hydroxide der Alkalimetalle Lithium, Natrium, Kalium und Caesium sowie der Erdalkalimetalle Magnesium, Calcium, Barium und Strontium, weiterhin Sulfate der Alkalimetalle und von Magnesium und Calcium, schließlich Carbonate der Alkalimetallverbindungen sowie Magnesiumcarbonat. Außerdem kommen Phosphate wie Natriumphosphat, Arsenate wie Ammonium-, Natrium- und Kaliumarsenat, Bleiverbindungen wie Bleiacetat, Bleichlorid und Bleinitrat, Bismutverbindungen wie Bismutnitrat und Bismutoxychlorid, Zinnverbindungen wie Zinnchlorid oder Alkalimetallstannate sowie Antimonverbindungen wie Ammonium-, Natrium- und Kaliumantimonat.

Die Konzentration der Übergangsmetallsalz- bzw. -sollösungen richtet sich zum einen nach der Löslichkeit der entsprechenden Verbindung. Sie sollte in der Regel mindestens 0,1 g/l und kann bis zur Sättigung der Lösung betragen. In der Regel enthalten die Lösungen 0,01 bis 5 Gew.-% an Übergangsmetallionen. Weiterhin richtet sich die Menge der aktiven Komponente nach ihrer im erfindungsgemäßen Trägerkatalysator gewünschten Konzentration. Die Lösungen werden im allgemeinen bei Raumtemperatur hergestellt.

### Verfahrensschritt b)

Die oben beschriebenen Lösungen der aktiven Komponenten oder ihrer Vorläuferverbindungen werden mit einem organischen Polymeren versetzt, wobei entweder die Lösung zum Polymer oder das Polymer zur Lösung gegeben werden können.

Das organische Polymer ist in der Lage, mindestens das Zehnfache seines Eigengewichts an Wasser zu binden. Solche Verbindungen werden als Hydrogele bezeichnet (vgl. B.D. Rathmer et al. in "Hydrogels for Medical and related Applications", ACS Symposium Series No. 31 (1976)). Es handelt sich hierbei um vernetzte polymere Verbindungen, wobei die Vernetzung durch ionische Wechselwirkungen oder Wasserstoffbrückenbindungen sowie durch chemische Vernetzung erfolgen kann. Es kommen z.B. Propfcopolymerisate aus Stärke und Acrylnitril (z.B. G.F. Fanta et al. in Starch 34 (1982) 95), Stärke und Acrylsäure (EP-A 83 022), Polysacchariden und Acrylsäure (DE-A 41 05 000), Copolymerisate aus Polyvinylalkohol und Natriumacrylaten (US-A 4 155 893), Copolymerisate aus Acrylamid und Acrylsäure (EP-A 72 214), vernetztes Polyethylenoxid (US-A 3 264 202), vernetztes Polyacrylamid (US-A 3 669 103), vernetztes Poly-N-Vinylpyrrolidon (US-A 3 669 103), vernetzte Polyvinylalkohole (Walter et al., Biomaterials 9 (1988) 150), vernetzte Carboxycellulosefasern (US-A 3 826 711), Hydrolysate von Polyvinylacetat-Acrylsäure-Copolymerisaten (GB 20 30 990) und Hydrolysate von Polyacrylnitril (US-A 4 366 206) in Betracht.

Bevorzugt werden vernetzte Polymere aus Acrylsäure, Acrylsäure und Acrylamid sowie aus Acrylamid. Besonders bevorzugt sind teilweise neutralisierte Natriumpolyacrylate, die schwach vernetzt sind. Als chemische Vernetzer kommen beispielsweise Diole wie Ethylenglykol, Polyethylenglykol und Polyole, Diamine, Diene in Mengen von 0,1 bis 5 Gew.-%, bezogen auf das Polymer, in Frage. Solche Polymere können bis zum 1000fachen ihres Eigengewichts an Wasser binden. Sie werden üblicherweise durch radikalische Polymerisation in wäßriger Lösung hergestellt und sind als Verdickungsmittel oder als sogenannte Superabsorber im Handel erhältlich (F.L. Buchholz, "Preparation and Structure of Polyacrylates" in Absorbent Polymer Technology, Studies in Polymer Science 8, Elsevier, Amsterdam 1990, S. 23).

Im allgemeinen wird das Polymer mit soviel Lösung der aktiven Komponente versetzt, daß es diese vollständig aufnehmen kann. Dabei quillt das Polymer auf. Dieser Vorgang ist im allgemeinen in 60 Minuten beendet. Das Quellen des Polymers wird üblicherweise bei Raumtemperatur vorgenommen. Beim Quellen von Polyacrylaten sollte der pH-Wert mindestens 6 betragen, da andernfalls nur eine unzureichende Lösungsaufnahme erfolgt.

### Verfahrensschritt c)

Das gequollene Polymer wird mit einem pulverförmigen Katalysator-Trägermaterial vermischt, wobei es keine Rolle spielt, in welcher Reihenfolge die Komponenten zueinander gegeben werden.

Als Trägermaterial kommen unter den Reaktionsbedingungen der zu katalysierenden Reaktion inerte Verbindungen in Betracht, bevorzugt Aluminiumoxide, Siliciumdioxid, Kieselgur, Kieselgel, Tonerden, z.B. Montmorillonite, Silikate, Zeolithe im Gemisch mit Aluminiumoxid, Zirkoniumoxide, Titanoxide sowie Gemische dieser Verbindungen untereinander, von denen Aluminiumoxid und Siliciumdioxid besonders bevorzugt sind.

Weiterhin können auch Oxide von Mg, Ca, Sr, Ba, Sulfate von Ca, Ba, Sr, Pb, Carbonate von Mg, Ca, Sr, Ba, Ni, Co, Mn, Fe, Cu, Sulfide von Mo, W, Co, Ni, Fe, Pb, Ag, Cr, Cu, Cd, Sn, Zn, Carbide von B, Si, W und Nitride von B, Si verwendet werden.

Die Menge des Trägermaterials beträgt im allgemeinen das 10- bis 1000fache, bevorzugt das 20- bis 200fache des nicht gequollenen Polymers.

Der Lösung können übliche Peptisierungsmittel zur Verbesserung der mechanischen Stabilität der erhaltenen Formkörper zugesetzt werden, wie Ammoniak für Aluminiumoxid-Trägermaterialien und Natronlauge für Siliciumdioxid. Die Menge beträgt in der Regel 0,1 bis 5 Gew.-%, bezogen auf das Trägermaterial.

Die Komponenten werden vermischt, wozu Kneter oder Mix-Muller benutzt werden können.

### Verfahrensschritt d)

Die weiteren Verarbeitungsmaßnahmen sind dem Fachmann an sich bekannt. Die nach Verfahrensschritt c) erhaltene Masse wird geformt, z.B. durch Extrusion im Extruder oder durch Verformen in einer Strangpresse zu Strängen mit den gewünschten Abmessungen.

Die so erhaltenen Formkörper werden getrocknet, wozu in der Regel Temperaturen von 100 - 150°C über 2 bis 24 h angewendet werden.

Anschließend werden die Formkörper im allgemeinen über 2 bis 24 h bei 300 bis 800°C, vorzugsweise 300 bis 550°C, calciniert, wobei die Polymeren aus der Trägermatrix entfernt werden und die thermisch labilen Salze der aktiven Komponenten in die Oxide, Mischoxide oder Halogenide überführt werden. Je nach aktiver Komponente kann sich daran ein Aktivierungsschritt anschließen, wobei die katalytisch aktive Komponente erst gebildet wird. Bei Hydrierkatalysatoren erfolgt dies z.B. durch eine Wasserstoffbehandlung bei Temperaturen von 80 bis 400°C unter meist drucklosen Bedingungen im Wasserstoffstrom.

Der Trockenschritt kann auch ausgelassen werden, jedoch hat es sich gezeigt, daß er für eine schonende Entfernung des Lösungsmittels vorteilhaft ist.

Die so erhaltenen Trägerkatalysatoren können weiterhin in an sich bekannter Weise auf nicht poröse Träger aus beispielsweise Steatit, auf Glasringe, Quarzringe oder hochgesinterte Aluminiumoxidringe aufgebracht werden. Dazu werden in der Regel die nichtporösen Träger mit gemahlenen Trägerkatalysatorpartikeln sowie einer Granulierflüssigkeit versetzt. Bei dieser Flüssigkeit kann es sich um Aluminiumnitrat-, Aluminiumacetat- oder Aluminiumnatriumhydroxidlösungen handeln, die nach einer Trocknung und Calcinierung der beschichteten nichtporösen Träger Feststoffbrücken zwischen den nichtporösen Trägern und den katalytischen Partikeln bilden.

Die erfindungsgemäßen Trägerkatalysatoren sind hochporös und weisen ein niedriges Schüttgewicht aus. Durch elektronenmikroskopische Aufnahmen ist deutlich erkennbar, daß sich der überwiegende Teil, in der Regel mehr als 80 %, der aktiven Komponenten in den Makroporen befindet. Eine Bestimmung des Anteils der Aktivkomponente, der sich in den Makroporen befindet, ist nur durch die Auswertung von mehreren repräsentativen Schnitten durch den Katalysatorstrang möglich, wobei bei der Rasterelektronenmikroskopie die schweren Elemente mit Hilfe der Rückstreuelektronen sichtbar gemacht werden.

Die Reaktionspartner können in den erfindungsgemäßen Katalysatoren die aktiven Zentren leicht erreichen und die Reaktionsprodukte können leicht abgeführt werden. Durch die Anordnung der aktiven Komponenten in den Makroporen ist es möglich, Katalysatoren herzustellen, die bei gleicher Aktivität, wie sie konventionelle Katalysatoren aufweisen, nur einen Bruchteil der Menge an aktiver Komponente erfordern.

Die erfindungsgemäßen Trägerkatalysatoren sind insbesondere für Hydrierungen, vor allem solche in der Flüssigphase geeignet. Beispiele dafür sind:
(in Klammern sind die jeweiligen bevorzugten aktiven Komponenten)
- die selektive Hydrierung von mehrfach ungesättigten Kohlenwasserstoffen in C₂- bis C₁₀-Kohlenwasserstoffströmen, die aus katalytischen oder thermischen Crack- oder Pyrolyseprozessen in Raffinerien oder Steamcrackern entstehen, insbesondere
   - die Hydrierung von Acetylen im C₂-Strom (Pd, Pt, Cu),
   - die Hydrierung von Methylacetylen und Propadien im C₃-Strom (Pd, Pt, Cu, Ag),
   - die Hydrierung von Butadien, Butin und Vinylacetylen im C₄-Strom (Pd, Pt, Cu),
   - die Hydrierung von Cyclopentadien, Pentadien und Isopren im C₅-Strom (Pd, Pt, Cu, Ni),
   - die Hydrierung von Dienen und Styrol im C₅-C₁₀-Strom (Pyrolysebenzin) (Pd, Pt, Cu, Ni),
- die Hydroraffination langkettiger Kohlenwasserstoffe aus Raffinerieströmen, insbesondere
   - der hydrierende Abbau von Sauerstoff, Schwefel, Stickstoff und aromatischen Verbindungen in Leichtbenzin, Schwerbenzin, Gasölen, Vakuumgasölen, Rückstandsölen, besonders die hydrierende Raffination von solventraffinierten und entparaffinierten Vakuumgasölen zu technischen und medizinischen Weißölen (Ni, Mo, W, Pt, Pd) und die hydrierende Raffination von Paraffinen (Ni, Mo, W, Pt, Pd),
   - die Hydrierung von Speisefetten (Fetthärtung) (Ni, Pd, Pt),
- Selektive Hydrierungen von funktionellen Gruppen in Polymeren, insbesondere die Hydrierung der olefinischen Doppelbindungen in Styrol-Butadien-Copolymeren (Ni, Pd, Pt),
- Isomerisierungen, insbesondere die Skelettisomerisierung von n-Butan zu iso-Butan (Pd, Pt),
- Dehydrierungen von C3-C15-Kohlenwasserstoffströmen mit Pd- oder Cr-haltigen Katalysatoren, insbesondere
   - die Dehydrierung von iso-Butan
   - die Dehydrierung von Butan zu Butenen und Butadien
   - die Dehydrierung von Propan zu Propen
   - die Dehydrierung von iso-Pentan zu Isopren
   - die Dehydrierung von C₆-C₁₅-Paraffinen,
- Selektive Oxidationen, insbesondere
   - die selektive Oxidation von Ethylen zu Ethylenoxid (Ag, Cu)
   - die selektive Oxidation von Ethylenglykol zu Glyoxal (Ag, Cu)

Bei Dehydrierungen zeichnen sich die erfindungsgemäßen Trägerkatalysatoren, bedingt durch den schnelleren Stoffaustausch in den Makroporen und der damit verbundenen geringeren Verweilzeit an der Oberfläche, durch eine geringere Verkokungsneigung bzw. längere Standzeiten aus (vgl. A. Wheeler, Adv. Catal. 3 (1951) 317).

### Beispiele

### Beispiel 1

### Herstellung eines erfindungsgemäßen Trägerkatalysators (Pd auf Al₂O₃)

5,47 g einer wäßrigen Palladiumnitratlösung (11 Gew.-% an Pd) wurden mit 150 ml Wasser und 3 g eines hochmolekularen Natriumpolyacrylats (90 % der Säuregruppen neutralisiert, vernetzt mit 0,4 mol-% Polyethylenglykol der Molmasse 1500), das das 300fache seines Eigengewichts an Wasser bindet, versetzt. Nach 0,5 h wurde die gelartige Masse mit 280 g Aluminiumoxid (Pseudoböhmit, Oberfläche nach Calcinieren bei 600°C: 300 m²/g) verknetet. Nach Zugabe von 200 ml Ammoniaklösung (enthaltend 50 ml konz. Ammoniak) wurde 1 h geknetet. Die Masse wurde bei 65 bar in einer Strangpresse zu 3,8 mm Strängen verformt, 16 h bei 120°C getrocknet und 6 h bei 330°C calciniert.

Analysedaten des Katalysators:

| | |
|---|---|
| Litergewicht | 383 g/l |
| BET-Oberfläche | 298 m²/mg |
| Porenvolumen (DIN 66 132) | 1,17 ml/g |
| (bestimmt durch Quecksilberporosimetrie, J. v. Brakel et al., Powder Technology 29 (1991) 1) | |

Mikroporen haben einen Durchmesser von < 20 nm, Mesoporen von 20 bis 100 nm und Makroporen von >100 nm.

Der erfindungsgemäße Katalysator wies folgende Analysedaten auf:
mittlerer Durchmesser der Makroporen [nm]: 600
mittlerer Durchmesser der Mikro- und Mesoporen [nm]: 4
Makroporenanteil [Vol.-%]: 33

### Beispiel 2

### Herstellung eines Vergleichskatalysators (Pd auf Al₂O₃)

3 g eines hochmolekularen Natriumpolyacrylats (90 % der Säuregruppen neutralisiert, vernetzt mit 0,4 % Polyethylenglykol der Molmasse 1500), das das 300fache seines Eigengewichts an Wasser bindet, wurden mit 280 g Pseudoböhmit im Kneter vermischt (Pulver A).

5,47 g einer wäßrigen Palladiumnitratlösung (11 Gew.-% an Pd) wurden in 150 ml Wasser gelöst. Diese Pd-haltige Lösung wurde zum Pulver A zugegeben. Nach Zugabe von 200 ml Ammoniaklösung (enthaltend 50 ml konz. Ammoniak) wurde 1 h geknetet. Die Masse wurde in einer Strangpresse zu 3,8 mm Strängen verformt, 16 h bei 120°C getrocknet und 6 h bei 330°C calziniert.

Beispiel 1 und 2 unterscheiden sich nur dadurch, daß die Pd-Nitratlösung in Beispiel 1 zum Vorquellen des Polymers verwendet wurde. In Beispiel 2 wurde die wäßrige Pd-Nitratlösung mit Pseudoböhmit und dem Polymer vermischt.

Analysedaten des Katalysators nach Beispiel 2:
Litergewicht [g/l]: 376
BET-Oberfläche [m²/g]: 276
Porenvolumen (DIN 66 132) [ml/g]: 1,09
bestimmt durch Quecksilberporosimetrie (J. v. Brakel et al., Powder Technology 29 (1991) 1)
mittlerer Durchmesser der Makroporen [nm]: 500
mittlerer Durchmesser der Mikro- und Mesoporen [nm]: 4,5
Makroporenanteil [Vol.-%]: 35

Ein Vergleich von elektronenmikroskopischen Aufnahmen an Schnitten durch die gemäß Beispiel 1 und 2 hergestellten Katalysatoren zeigt, daß sich im erfindungsgemäßen Katalysator die Aktivkomponente zum Großteil (> 80 %) in den Makroporen befindet. Im Vergleichskatalysator dagegen befindet sich die Aktivkomponente überwiegend in den Mikroporen und nur zu ca. 10 % in den Makroporen.

### Beispiel 3

### Verwendung der Trägerkatalysatoren nach den Beispielen 1 und 2 zur selektiven Hydrierung eines C₄-Stroms

Ein C₄-Strom gemäß der folgenden Tabelle wurde an den Katalysatoren nach den Beispielen 1 und 2 in Rieselfahrweise bei 50°C und 14,1 bar Druck bei einer Raum-Zeit-Geschwindigkeit, bezogen auf den C₄-Strom, von 6,4 1/1 x h umgesetzt.

Die Katalysatoren wurden vor der Hydrierung im Wasserstoffstrom (drucklos, 150°C, 8 h, 20 l H₂/l · h) reduziert.

Die folgende Tabelle zeigt die Zusammensetzung des C4-Stroms sowie des Hydrierproduktes:

| Zusammensetzung in mol.-% | | | |
|---|---|---|---|
| | C₄-Strom | Hydrierprodukt | |
| | | Beispiel 1 | Beispiel 2 |
| Butadien | 45,6 | 3,2 | 3,3 |
| Buten-1 | 15,9 | 38,7 | 36,6 |
| trans-Buten-2 | 4,9 | 20,2 | 20,1 |
| cis-Buten-2 | 3,7 | 7,3 | 7,5 |
| iso-Buten | 24,2 | 24,2 | 24,2 |
| iso-Butan | 1,5 | 1,5 | 1,5 |
| n-Butan | 4,2 | 4,9 | 6,8 |

Der erfindungsgemäße Katalysator hydriert Butadien deutlich selektiver zu Butenen, was an der deutlich geringeren n-Butanbildung erkennbar ist.

An einem konventionellen Katalysator (0,3 Gew.-% Pd auf α-Al₂O₃, Litergewicht 1100 g/l) erhält man bei gleichem Butadienumsatz eine geringere Selektivität (n-Butan-Bildung ca. 1 %). Der Vorteil des erfindungsgemäßen Katalysators besteht weiterhin aufgrund seines geringen Litergewichts in einer um den Faktor 2,8 höheren Raumgeschwindigkeit (bezogen auf die Katalysatormasse) und eines um den Faktor 2,8 kleineren Gehalts an aktiver Komponente.

### Beispiel 4

### Herstellung eines Pd/Ag-Schalenkatalysators

3,72 g einer wäßrigen Palladiumnitratlösung (11 Gew.-% an Pd) und 6,44 g Silbernitrat wurden in 150 ml verdünntem Ammoniak gelöst. Diese Pd- und Ag-haltige Lösung wurde zu 6 g eines hochmolekularen Natriumpolyacrylats (90 % der Säuregruppen neutralisiert, vernetzt mit 0,4 % Polyethylenglykol der Molmasse 1500), das das 300fache seines Eigengewichts an Wasser binden kann, gegeben.

Nach 1 h wurde die gelartige Masse mit 280 g Pseudoböhmit (Oberfläche nach Calzinieren bei 600°C: 300 m²/g) verknetet. Nach Zugabe von insgesamt 175 ml Wasser wurde 1 h geknetet. Die Masse wurde in einer Strangpresse bei einem Preßdruck von 60 bar zu 4,0 mm Strängen verformt, 16 h bei 120°C getrocknet und 4 h bei 400°C calziniert.

Die calzinierten Stränge wurden in einer Kugelmühle gemahlen. Steatitkugeln (Durchmesser 4,3 - 4,7 mm) wurden mit diesem Material beschichtet. Als Granulierflüssigkeit wurde eine 4,2 prozentige Aluminiumnitrat-Lösung verwendet. Die beschichteten Kugeln wurden 16 h bei 90°C getrocknet und 3 h bei 530°C calziniert.

Die fertigen Kugeln wiesen einen Pd-Gehalt von 0,02 Gew.-% und einen Ag-Gehalt von 0,2 Gew.-% auf. Mit dieser Methode wird ein Katalysator erhalten, der die Aktivkomponenten in einer makroporösen Schale trägt, wobei die Aktivkomponenten in den Makroporen der Schale angereichert sind.

### Beispiel 5

### Verwendung des unter Beispiel 4 beschriebenen Katalysators zur selektiven Hydrierung von Acetylen im C₂-Strom

Der unter Beispiel 4 beschriebene Pd/Ag-Schalenkatalysator wurde für die selektive Gasphasen-Hydrierung von Acetylen im C₂-Strom eingesetzt.

Ein Reaktor mit einem Katalysatorvolumen von 66 ml wurde mit 200 l/h eines C₂-Stroms (Zusammensetzung: 99 Vol.-% Ethylen, 1 % Acetylen) und 2,2 l/h Wasserstoff beschickt. Bei einem Druck von 20 bar und einer Temperatur am Reaktoreingang von 31°C wurde ein Acetylen-Umsatz von 90 % bei einer Selektivität von 78 % erzielt.

### Beispiel 6

### Verwendung des erfindungsgemäßen Katalysators nach Beispiel 1 für die selektive Hydrierung von Methylacetylen und Propadien in einem C₃-Strom in der Flüssigphase

Ein C₃-Strom gemäß der folgenden Tabelle wurde am erfindungsgemäßen Katalysator nach Beispiel 1 in Sumpffahrweise bei einer Eintrittstemperatur von 4°C und 20 bar und einer Belastung, bezogen auf den C₃-Strom, von 9,5 kg/l*h umgesetzt. Das molare Verhältnis von Wasserstoff zu Methylacetylen/Propadien betrug 1,1:1.

Der nach Beispiel 1 hergestellte Katalysator wurde vor der Hydrierung im Wasserstoffstrom (drucklos, 150°C, 3 h, 20 l H₂/l*h) vorreduziert.

Der Gehalt an Methylacetylen/Propadien wurde von 4,07 % auf < 140 ppm erniedrigt.

| Zusammensetzung in mol.-% | | |
|---|---|---|
| | C₃-Strom | Hydrierprodukt |
| Methylacetylen | 2,32 | < 20 ppm |
| Propadien | 1,75 | 120 ppm |
| Propen | 90,78 | 93,69 |
| Propan | 5,13 | 5,8 |
| Unbekannte Verbindungen | 0,02 | 0,5 |

## Patentansprüche

1. Trägerkatalysatoren, bei denen sich der überwiegende Teil der aktiven Komponenten in den Makroporen mit einem Durchmesser oberhalb von 100 nm befindet, erhältlich durch
a) Lösen einer katalytisch aktiven Komponente oder deren Vorläuferverbindung in einem Lösungsmittel,
b) Versetzen der so erhaltenen Lösung mit einem organischen Polymeren, das in der Lage ist, mindestens das Zehnfache seines Eigengewichts an Wasser zu binden, so daß das Polymer unter Quellung diese Lösung vollständig aufnimmt,
c) anschließendes Vermischen des Polymeren mit einem Katalysator-Trägermaterial,
d) Formen der so erhaltenen Masse, Trocknen und Calcinieren.

2. Trägerkatalysatoren nach Anspruch 1, zu deren Herstellung man Wasser oder ein Gemisch aus Wasser und Ammoniak als Lösungsmittel verwendet.

3. Trägerkatalysatoren nach Anspruch 1 oder 2, zu deren Herstellung man vernetzte Natriumpolyacrylate als organisches Polymer verwendet.

4. Trägerkatalysatoren nach den Ansprüchen 1 bis 3, zu deren Herstellung man wasserlösliche Palladiumsalze als Vorläuferverbindung für die aktive Komponente verwendet.

5. Verfahren zur Herstellung von Trägerkatalysatoren gemäß den Ansprüchen 1 bis 4, dadurch gekennzeichnet, daß man
a) eine katalytisch aktive Komponente oder deren Vorläuferverbindung in einem Lösungsmittel löst,
b) die so erhaltene Lösung mit einem organischen Polymeren versetzt, das in der Lage ist, mindestens das Zehnfache seines Eigengewichts an Wasser zu binden,
c) das Polymer mit einem Katalysator-Trägermaterial vermischt,
d) die so erhaltene Masse formt, trocknet und calciniert.

6. Verwendung von Trägerkatalysatoren gemäß Anspruch 4 zur selektiven Hydrierung ungesättigter organischer Verbindungen.

7. Verfahren zur selektiven Hydrierung von Butin, Butadien oder Gemischen dieser Verbindungen zu Buten, dadurch gekennzeichnet, daß man die Hydrierung an einem Trägerkatalysator gemäß Anspruch 1 durchführt.

8. Verfahren zur selektiven Hydrierung von Acetylen zu Ethylen, dadurch gekennzeichnet, daß man die Hydrierung an einem Trägerkatalysator gemäß Anspruch 1 durchführt.

9. Verfahren zur selektiven Hydrierung von Methylacetylen, Propadien oder Gemischen dieser Verbindungen zu Propen, dadurch gekennzeichnet, daß man die Hydrierung an einem Trägerkatalysator gemäß Anspruch 1 durchführt.

## Claims

1. A supported catalyst, in which the predominant part of the active components is present in the macropores having a diameter above 100 nm, obtainable by
a) dissolving a catalytically active component or its intermediate in a solvent,
b) adding an organic polymer which is capable of binding at least ten times its own weight of water to the solution thus obtained, so that, with swelling, the polymer completely absorbs this solution,
c) then mixing the polymer with a catalyst carrier,
d) molding the material thus obtained, drying and calcining.

2. A supported catalyst as claimed in claim 1, for the preparation of which water or a mixture of water and ammonia is used as the solvent.

3. A supported catalyst as claimed in claim 1 or 2, for the preparation of which a crosslinked sodium polyacrylate is used as the organic polymer.

4. A supported catalyst as claimed in any of claims 1 to 3, for the preparation of which a water-soluble palladium salt is used as an intermediate for the active component.

5. A process for the preparation of a supported catalyst as claimed in any of claims 1 to 4, wherein
a) a catalytically active component or its intermediate is dissolved in a solvent,
b) an organic polymer which is capable of binding at least ten times its own weight of water is added to the solution thus obtained,
c) the polymer is mixed with a catalyst carrier,
d) the material thus obtained is molded, dried and calcined.

6. Use of a supported catalyst as claimed in claim 4 for the selective hydrogenation of unsaturated organic compounds.

7. A process for the selective hydrogenation of butyne, butadiene or mixtures of these compounds to butene, wherein the hydrogenation is carried out over a supported catalyst as claimed in claim 1.

8. A process for the selective hydrogenation of acetylene to ethylene, wherein the hydrogenation is carried out over a supported catalyst as claimed in claim 1.

9. A process for the selective hydrogenation of methylacetylene, propadiene or mixtures of these compounds to propene, wherein the hydrogenation is carried out over a supported catalyst as claimed in claim 1.

## Revendications

1. Catalyseurs sur supports dans lesquels la plus grande partie des composants actifs se trouve dans les macropores de diamètre supérieur à 100 nm, et qu'on obtient
a) en dissolvant un composant à activité catalytique ou son composé précurseur dans un solvant,
b) en ajoutant à la solution ainsi obtenue un polymère organique capable de fixer au moins dix fois son poids d'eau, de sorte que le polymère absorbe totalement cette solution en gonflant,
c) en mélangeant ensuite le polymère avec une matière de support pour catalyseur,
d) en façonnant la masse obtenue, en la séchant et en la calcinant.

2. Catalyseurs sur supports selon la revendication 1, à la préparation desquels on a utilisé, en tant que solvant, l'eau ou un mélange d'eau et d'ammoniaque.

3. Catalyseurs sur supports selon la revendication 1 ou 2, à la préparation desquels on a utilisé, en tant que polymère organique, un polyacrylate de sodium réticulé.

4. Catalyseurs sur supports selon les revendications 1 à 3, à la préparation desquels on a utilisé, en tant que composé précurseur du composant actif, un sel de palladium soluble dans l'eau.

5. Procédé pour la préparation de catalyseurs sur supports selon les revendications 1 à 4, caractérisé par le fait que
a) on dissout un composant à activité catalytique ou son composé précurseur dans un solvant,
b) on ajoute à la solution obtenue un polymère organique capable de fixer au moins dix fois son poids d'eau,
c) on mélange le polymère avec une matière de support pour catalyseurs,
d) on façonne la masse obtenue, on la sèche et on la calcine.

6. Utilisation des catalyseurs sur supports selon la revendication 4 pour l'hydrogénation sélective de composés organiques insaturés.

7. Procédé pour l'hydrogénation sélective du butyne, du butadiène ou de mélanges de ces composés en butène, caractérisé par le fait que l'on procède à l'hydrogénation sur un catalyseur sur support selon la revendication 1.

8. Procédé pour l'hydrogénation sélective de l'acétylène en éthylène, caractérisé par le fait que l'on procède à l'hydrogénation sur un catalyseur sur support selon la revendication 1.

9. Procédé pour l'hydrogénation sélective du méthylacétylène, du propadiène ou d'un mélange de ces composés en propène, caractérisé par le fait que l'on procède à l'hydrogénation sur un catalyseur sur support selon la revendication 1.
